# EUROPEAN PATENT SPECIFICATION

(11) **EP 0 558 525 B1**
(45) Date of publication and mention of the grant of the patent: **28.12.1994**
(21) Application number: 91919504.0
(22) Date of filing: 17.10.1991
(51) Int. Cl.: A23K 1/00

(54) **SEMDURAMICIN PREMIX**
SEMDURAMICIN ENTHALTENDE TIERFUTTERVORMISCHUNG
PREMELANGE POUR SEMDURAMICINE

(30) Priority: 16.11.1990 US 614365
(43) Date of publication of application: 08.09.1993
(73) Proprietor: PFIZER INC., New York, N.Y. 10017 (US)
(72) Inventor: GRIZZUTI, Antonio, Old Lyme, CT 06371 (US); LLOYD, Robert, Joseph, Mystic, CT 06355 (US)
(74) Representative: Moore, James William, Dr.
(86) International application number: PCT/US91/07498
(87) International publication number: WO 92/08373

(56) References cited:
- EP-A- 0 060 680
- EP-A- 0 171 628
- EP-A- 0 272 119
- GB-A- 1 030 297
- GB-A- 1 572 114
- US-A- 3 947 586
- US-A- 4 804 680

## Description

### Technical Field

The field of art to which this invention pertains is animal premixes and particularly, Semduramicin premixes.

### Background of the Invention

Many animal drugs are administered by admixture with the animal feed. Typically, to facilitate a uniform drug-feed mixture a drug-feed premix is prepared because of the very low concentration of drug to feed used. The concentrated drug premix is added to and mixed through batches of feed.

Premixes are characterized by a variety of associated properties such as stability, flowability, and dustiness. Typical premixes represent a compromise of the above properties, as for example, an increase in flowability may adversely affect the dustiness of the premix.

EP 0272119 discloses a stable livestock feed premix composition containing acid-sensitive antibiotic esters and a method for stabilizing an acid-sensitive antibiotic ester by incorporating said ester into a feed premix composition. The claims are specifically directed to composition of "laidlomycin."

EP 0171628 discloses ionophore polyether antibiotic anticoccidial premix compositions comprising 0.25% to 35% on a weight basis of an ionophore polyetherantiobiotic, 0.75% to 35% of a physiologically acceptable alcohol such as benzyl alcohol, phenethyl alcohol and propylene glycol, 0.0% to 10.0% of a vegetable oil such as corn oil, or additional propylene glycol, and 30.0% to 99.00% of a sorptive, edible organic carrier such as corncob grits, extracted cornmeal, expanded corn grits, solvent extracted soybean meal, sorghum, or wheat middlings and the like or a sorptive silica or a silicate.

EP 0060680 discloses a process for preparing a premix composition comprising a synthetic drug, carrier and binder for the purpose of reducing carryover of the synthetic drug to subsequent lots of animal feed.

Although there are a variety of premixes there is a continual search in this field of art for premixes that exhibit an improved mix of properties.

### Summary of the Invention

This invention is directed to an animal premix having improved levels of flowability and dustiness. The premix comprises 2% to 10% Semduramicin or its pharmaceutically acceptable cationic salts thereof, 0.5% to 50% Semduramicin degradation reducing stabilizer, 40% to 80% diluent, 5% to about 50% density-increasing bulking agent, 2% to 10% dust controlling oil and 0.25% to 5% flowability enhancing glidant selected from the group consisting of sodium aluminosilicate and silicon dioxide.

The invention is also directed to an animal feed containing the above described premix.

Other features and advantages will be apparent from the specification and claims which describe an embodiment of this invention.

### Detailed Description of the Invention

Although this invention is directed to a premix for Semduramicin (i.e. UK-61,689; an antibiotic) or its pharmaceutically acceptable cationic salts thereof (hereinafter referred to as Semduramicin) other beneficial agents (e.g. drugs) may be substituted for Semduramicin provided that the resulting formulation has the desired flowability, stability and lack of dustiness. Preferred cationic salts are the sodium, potassium and ammonium salts. An especially preferred salt is the sodium salt. Semduramicin and its production are described in U.S. Pat. No. 4,804,680.

Semduramicin is active against a variety of microorganisms and is effective in controlling coccidiosis, enteritis and swine dysentery as well as being effective in promotion of growth and/or improving efficiency of feed utilization in swine and ruminants.

Any amount of Semduramicin may be used in the premix that provides the desired efficacy, for the above described applications, when the premix is mixed with feed and fed to the animal. However, typically, the Semduramicin will be present in an amount from 2 to 10% by weight of total premix. (Where used herein, the "%" symbol is meant to define percent by weight.) The preferred amount is 5 to 7% by weight. These amounts have been shown to be efficacious when administered to animals in the conventional feedmix of 0.45 g (1 pound) premix to 900 kg (1 ton) feed. The especially preferred use level in chicken feed is generally in the range of 15 to 120 ppm. A typical Semduramicin particle size is from 5 to 100 micron.

Typically a fine particle stabilizer (e.g. about 0.1 mm to about 0.8 mm) that is effective in substantially reducing the degradation (e.g. hydrolysis) of Semduramicin is added to the premix. Monovalent basic or neutral salts, for example sodium carbonate, sodium sulfate, ammonium hydroxide, ammonium carbonate, potassium carbonate and sodium phosphate are effective. Preferably sodium carbonate, sodium sulfate or sodium chloride is used. It is believed that the presence of the salt reduces the solubility of the Semduramicin (when present as a salt) through the common ion effect. In addition, materials that increase the alkalinity of the medium appear to increase the stability of the drug (e.g. sodium carbonate). Any amount of stabilizer may be used that is effective in stabilizing the Semduramicin. However, typically 0.5% to 50% stabilizer is added to the premix. Actually little advantage in stabilization is achieved with levels above 10%, and high levels of stabilizer may lead to insufficient quantities of other components. Below 0.5% the desired stability is typically not achieved. Preferably 3% to 6% stabilizer is added to the premix.

In order to achieve the desired predetermined premix concentration, a carrier (i.e. diluent) is typically used as a component of the premix. Typically the particle size is 0.1 to 0.9 mm. The desired premix concentration of Semduramicin depends on the desired rate of addition of premix to finished feed. A diluent is typically an edible substance used to mix with and reduce the concentration of nutrients and/or additives to make them more acceptable to animals, safer to use, and more capable of being mixed uniformly in a feed. Exemplary diluents are plant by-products however other suitable diluents include vermiculite, almond shells, rapeseed meal and limestone. The term by-products refers to secondary products that are produced in plant processing in addition to the principle product. Generally this means low cost, low nutritional, but edible materials. Preferred plant by-products are grain by-products and vegetable by-products. Preferable grain by-products diluent are soybean based, rice based, wheat based, and corn based. Especially preferred diluents are soybean mill run, soybean meal, soybean hulls, soybean grits, rice hulls, rice bran, rice husks, wheat bran, wheat middlings, wheat meal, wheat germ, corn cob, corn meal, corn gluten, corn cob grits and corn germ meal. Typically from 40% to 80% diluent is used. However it is preferred that from 40% to 60% diluent is used because below about 40% an undesirable quantity of the below described bulking agent may be required and above 60% the premix density may be too low. It is especially preferred that 45% to 55% diluent is used.

An amount of fine particle bulking agent (e.g. 0.1 mm to 0.9 mm) effective to provide the premix with bulk density of 0.5 to 0.8 g/ml (30 to 50 lbs/ft³) is added to the premix. Because of the low density of for example, the diluent, the bulking agent increases the density to the desired commercial level. Typical bulking agents have a density of 2.5 g/ml to 3.0 g/ml. Exemplary bulking agents are inert, high density materials (e.g. inert minerals, salts). Preferred bulking agents are limestone, sodium carbonate, kaolin, bentonite, oyster shells and sodium sulfate. Typically 5% to 50% bulking is added to the premix, however it is preferred to add 30% to 40% bulking agent because below about 30% the premix density may be too low.

An amount of oil effective to control dust is added to the premix. Generally it is preferred to have a dust (e.g. fine dry particulate matter) level that results in a safe, comfortable human environment during transferal of the premix. In this invention it is desired to reduce the levels of, in particular, Semduramicin dust. It is preferred to reduce the levels of Semduramicin dust to less than or equal to 100 micrograms per membrane and especially preferred to reduce the levels of Semduramicin dust to less than or equal to 25 micrograms per membrane. These levels are measured according to a standard dustiness test described below (prior to the Example section). Any oil may be used that is effective in achieving the desired dust levels and does not deleteriously effect other desired premix characteristics. Exemplary oils are petroleum oils ( e.g. mineral oils) and plant oils. In particular plant oils such as babassu oil, canola oil, castor oil, cocoa butter, coconut oil, corn oil, cotton seed oil, linseed oil, mustard oil, neem oil, niger-seed oil, oiticica oil, olive oil, palm oil, palm-kernel oil, peanut oil, perilla oil, poppy-seed oil, rapeseed oil, safflower oil, sesame oil, soybean oil, sunflower-seed oil, tung oil and wheat-germ oil may be used. Preferably mineral, soybean or rapeseed oil is used.

Preferably a mineral oil having a density between 0.7 grams per milliliter (g/ml) and 1.0 g/ml is used. It is preferred that a low density mineral oil is used when sodium carbonate is used as either the bulking agent or the stabilizer because this improves flowability. By low density is meant from 0.7 g/ml to 0.87 g/ml. It is also preferred that a high density mineral oil is used when limestone is used as a bulking agent because this improves flowability. By high density is meant greater than 0.87 g/ml to less than or equal to 1 g/ml. Preferably 2% to 10% oil is used because below 2% oil the level of dust may be undesirable and above 10% oil the flowability may be adversely effected (e.g. an undesirable amount of glidant (described below) may be necessary). It is especially preferred that 5% to 6.5% oil is used.

An amount of glidant effective to achieve the desired flowability, (greater than or equal to 8.5 g per second per cm² (0.12 pounds per second per square inch)), is added to the premix. This flowability level is determined according to a simple standard test described just prior to the Examples. Exemplary glidants are sodium aluminosilicate and silicon dioxide; however sodium aluminosilicate is preferred as it provides better flowability. A preferred form of silicon dioxide is colloidal silicon dioxide, which is submicroscopic fumed silica. It is light, non-gritty amorphous powder. A preferred form of sodium aluminosilicate is a hydrate having a particle size less than 0.015 cm (150 micron). Preferably 0.25% to 5% glidant is used as below 0.25% the flowability may not be sufficient and above 5% no additional advantage is gained, although more could be used without deleterious effect. It is especially preferred that 2% to 3% glidant is used. In addition, it is preferred that if 0.25% to 2% glidant is used, that less than 6.5% oil is used because that provides a better flow rate.

Some of the above ingredients inherently serve dual functions. Some components can function as a stabilizer and bulking agent (e.g. sodium carbonate). In the above description if a particular ingredient inherently performs more than one function the percentages are effected in the following manner. For a multi-use component, its percent in the formulation is added to each specific use, when totaling a percentage of a specific ingredient category. For example, if sodium carbonate is used at a 20% level, 20% would be considered as part of the total amount of stabilizer required and 20% would also be considered as contributing toward the total amount of bulking agent required.

The premixes of this invention may be made by any procedure that provides a premix having the desired properties of flow, bulk density, efficacy, stability, dust control and non-caking. However typically the solid ingredients (except for glidant) are mixed together and then the oil and glidant are mixed in, in succession. It is preferred that the glidant is added last as this facilitates the desired flowability described above. It is especially preferred that the carrier, bulking agent, and stabilizer are mixed together with one-half of the oil. The drug is then added, followed by the remainder of the oil and the glidant.

Typically these premixes are added to feed which is then feed to the animals requiring the Semduramicin. In general 0.23 to 0.9 kg (0.5 to 2lb) of premix is used for 900 kg (1 ton) of feed. Preferably 0.45 kg (1 lb.) of premix is used for 900 kg (1 ton)of feed. The feeds used are those which are useful for the animals that Semduramicin is an effective antibacterial or growth stimulating agent (e.g. swine, chickens).

Flowability levels as described herein are determined by reference to a standard test. The test comprises the flow of the premix through a funnel. The data measured in terms of grams per second per square centimetre and in pounds per seconds per square inches. The funnel used was constructed from stainless steel and had no welds or obstructions in the funnel path. The interior of the funnel was polished to a smooth finish. The funnel comprised a cylindrical portion 6.35 cm (2.5 inches) in diameter 15.2 cm (six inches) long. The cylindrical portion converged over a distance of 5.7 cm (2.25 inches to an interior diameter of 1.5 cm (0.6 inch). A cylindrical portion having a diameter of 1.5 cm (0.6 inch) extended from the converging funnel portion for a distance of 2.5 in (1 inch.)

The flowability test procedure follows. A sample of premix was placed into the metal funnel described above while keeping the bottom end closed using a dry finger. Using a stopwatch, the time required for the premix to completely flow through the funnel was measured. The stopwatch was started simultaneously with the removal of the finger from the funnel's bottom. The stopwatch was stopped when the powder flow from the funnel was completed. The density (unpacked) was determined by flowing the premix into a graduated cylinder using the above funnel. The premix's volume was read from the graduated cylinder.

Dustiness levels as described herein are determined by reference to a standard test. The dust is generated from the premix sample to be tested in a commercially available dust testing equipment (Heubach Dustmeter available from Heubach Engineering GmbH located in Germany). The generated dust was transported onto a filter membrane via an air stream. The content of active ingredient in the dust collected on the membrane was determined quantitatively by a suitable method. In brief the dust test apparatus comprised a rotating drum, of about two liters volume, into which the premix was placed. The rotating drum, at the downstream end, was in fluid communication with the bottom of a 1000 ml flask via a connection pipe approximately 23 cm (9 inches) long which fed through a hole in the bottom of the flask. The top of the flask was in fluid communication with a filter box of 17 cm² surface area. A suitable vacuum pump was connected to the upstream end of the filter box.

The dustiness test procedure follows. The premix sample was placed in the dust generating drum. The vacuum air flow rate was set at 4 liters/minute. The rotating drum was set for 30 revolutions per minute and the drum motor and vacuum pump were turned on for 5 minutes. After five minutes the test apparatus was automatically turned off. The filter membrane was removed from the filter holder and the drug was dissolved and assayed. Examples 1-9 detail data that shows the premix invention has satisfactory flowability and dustiness levels (according to the above described parameters). Examples 10-18 illustrate other premixes that did not have satisfactory levels of flowability and dustiness.

### EXAMPLE 1

A batch of medicated animal feed premix was prepared using the procedure described below.

The proportions of drug and excipients used for this batch are:

| | |
|---|---|
| Semduramicin Sodium | 5.64% |
| Rice Hulls | 48.86% |
| Limestone (calcium carbonate) | 33.0% |
| Sodium Carbonate | 4.0% |
| High Viscosity Mineral Oil | 6.5% |
| Sodium Aluminosilicate | 2.0% |

Manufacturing Procedure. The Procedure used for making the batches is as follows:
1. The CARRIER (rice hulls) was placed into a 2-liter beaker. The OIL was slowly added to the carrier in the beaker (using low pressure spray bottle). The carrier and oil were thoroughly mixed using a mechanical mixer (WAB model Turbula) for 10 minutes.
2. The BULKING AGENT (limestone), STABILIZER (sodium carbonate) and DRUG were added to the contents in the beaker from Step 1. The bulking agent, stabilizer, and drug were thoroughly mixed with the carrier and oil from Step 1 using a mechanical mixer (WAB model Turbula) for 15 minutes.
3. The GLIDANT was added to the mixture of carrier, oil, bulking agent, stabilizer, and drug from Step 2. This mixture, now containing all drug and excipients, was thoroughly mixed using a mechanical mixer (WAB model Turbula) for 10 minutes.
4. The completed medicated animal feed premix was placed into an appropriately sized bottle labeled with formulation and lot numbers. The flowability of the premix was determined using the funnel test (described elsewhere in this document).
   FLOWABILITY (metal funnel), 9.87 g/sec/cm² (0.141 lb/sec/in²): (initial)
   HEUBACH DUSTINESS VALUE (µg drug/membrane):
   not determined
The following Example premixes (2-19) were prepared in an analogous fashion to the premix preparation used in Example 1.

### EXAMPLE 2

| | |
|---|---|
| Semduramicin Sodium | 5.45% |
| Rice Hulls | 53.8% |
| Limestone (calcium carbonate) | 32.3% |
| Sodium Carbonate | 3.56% |
| High Viscosity Mineral Oil | 3.96% |
| Colloidal silicon dioxide | 0.99% |
| FLOWABILITY (metal funnel,) 10.71 g/sec/cm² (0.153 lb/sec/in²): (initial) HEUBACH DUSTINESS VALUE (µg drug/membrane): 72 | |

### EXAMPLE 3

| | |
|---|---|
| Semduramicin Sodium | 5.5% |
| Rice Hulls | 50.2% |
| Limestone (calcium carbonate) | 32.8% |
| Sodium Carbonate | 4.0% |
| High Viscosity Mineral Oil | 6.0% |
| Sodium Aluminosilicate | 1.5% |
| FLOWABILITY (metal funnel) 10.78 g/sec/cm², (0.154 lb/sec/in²) (initial) HEUBACH DUSTINESS VALUE (µg drug/membrane): 0.20 | |

### EXAMPLE 4

| | |
|---|---|
| Semduramicin Sodium | 5.5% |
| Rice Hulls | 49.7% |
| Limestone (calcium carbonate) | 32.5% |
| Sodium Carbonate | 3.9% |
| High Viscosity Mineral Oil | 5.9% |
| Sodium Aluminosilicate | 2.5% |
| FLOWABILITY (metal funnel) 11.55 g/sec/cm² (0.165 lb/sec/in²) (initial) HEUBACH DUSTINESS VALUE (µg drug/membrane): <0.1 | |

### EXAMPLE 5

| | |
|---|---|
| Semduramicin Sodium | 5.5% |
| Rice Hulls | 51.3% |
| Limestone (calcium carbonate) | 33.5% |
| Sodium Carbonate | 4.1% |
| High Viscosity Mineral Oil | 4.1% |
| Sodium Aluminosilicate | 1.5% |
| FLOWABILITY (metal funnel) 12.39 g/sec/cm² (0.177 lb/sec/in²) (initial) HEUBACH DUSTINESS VALUE (µg drug/membrane): 35.8 | |

### EXAMPLE 6

| | |
|---|---|
| Semduramicin Sodium | 5.5% |
| Rice Hulls | 50.5% |
| Limestone (calcium carbonate) | 33.0% |
| Sodium Carbonate | 4.0% |
| High Viscosity Mineral Oil | 5.0% |
| Sodium Aluminosilicate | 2.0% |
| FLOWABILITY (metal funnel), 11.27 g/sec/cm² (0.161 lb/sec/in²) (initial) HEUBACH DUSTINESS VALUE (µg drug/membrane): 17.3 | |

### EXAMPLE 7

| | |
|---|---|
| Semduramicin Sodium | 5.45% |
| Rice Hulls | 53.8% |
| Sodium Carbonate | 35.8% |
| Light Mineral Oil | 3.96% |
| Colloidal silicon dioxide | 0.99% |
| FLOWABILITY (metal funnel), 10.15 g/sec/cm² (0.145 lb/sec/in² (initial) HEUBACH DUSTINESS VALUE (µg drug/membrane): 92 | |

### EXAMPLE 8

| | |
|---|---|
| Semduramicin Sodium | 5.45% |
| Rice Hulls | 53.8% |
| Limestone (calcium carbonate) | 32.3% |
| Sodium Carbonate | 3.56% |
| High Viscosity Mineral Oil | 3.96% |
| Sodium Aluminosilicate | 0.99% |
| FLOWABILITY (metal funnel), 12.32 g/sec/cm² (0.176 lb/sec/in² (initial) HEUBACH DUSTINESS VALUE (µg drug/membrane): 19 | |

### EXAMPLE 9

| | |
|---|---|
| Semduramicin Sodium | 5.45% |
| Rice Hulls | 53.8% |
| Sodium Carbonate | 35.8% |
| Light Mineral Oil | 3.96% |
| Colloidal silicon dioxide | 0.99% |
| FLOWABILITY (metal funnel), 12.11 g/sec/cm² (0.173 lb/sec/in²) (initial) HEUBACH DUSTINESS VALUE (µg drug/membrane): 51 | |

### EXAMPLE 10

| | |
|---|---|
| Semduramicin Sodium | 5.5% |
| Soybean Millrun | 85.5% |
| Sodium Carbonate | 4.0% |
| High Viscosity Mineral Oil | 4.0% |
| Sodium Aluminosilicate | 1.0% |
| FLOWABILITY (metal funnel), 7.84 g/sec/cm² (0.112 lb/sec/in²) (1-week) HEUBACH DUSTINESS VALUE (µg drug/membrane): not determined | |

### EXAMPLE 11

| | |
|---|---|
| Semduramicin Sodium | 5.64% |
| Rice Hulls | 48.36% |
| Limestone (calcium carbonate) | 33.0% |
| Sodium Carbonate | 4.0% |
| High Viscosity Mineral Oil | 7.0% |
| Sodium Aluminosilicate | 2.0% |
| FLOWABILITY (metal funnel): no flow HEUBACH DUSTINESS VALUE (µg drug/membrane): not determined | |

### EXAMPLE 12

| | |
|---|---|
| Semduramicin Sodium | 5.5% |
| Rice Hulls | 56.7% |
| Limestone (calcium carbonate) | 34.0% |
| Sodium Carbonate | 3.78% |
| FLOWABILITY (metal funnel), 11.06 g/sec/cm² (0.158 lb/sec/in²) (3-day) HEUBACH DUSTINESS VALUE (µg drug/membrane): 2890 | |

### EXAMPLE 13

| | |
|---|---|
| Semduramicin Sodium | 5.5% |
| Rice Hulls | 55.5% |
| Limestone (calcium carbonate) | 33.3% |
| Sodium Carbonate | 3.7% |
| Light Mineral Oil | 2.0 |
| FLOWABILITY (metal funnel), 7.35 g/sec/cm² (0.105 lb/sec/in²) (3-day) HEUBACH DUSTINESS VALUE (µg drug/membrane): 610 | |

### EXAMPLE 14

| | |
|---|---|
| Semduramicin Sodium | 5.5% |
| Rice Hulls | 53.1% |
| Limestone (calcium carbonate) | 31.86% |
| Sodium Carbonate | 3.54% |
| Light Mineral Oil | 6.0% |
| FLOWABILITY (metal funnel); no flow initial HEUBACH DUSTINESS VALUE (µg drug/membrane): <0.1 | |

### EXAMPLE 15

| | |
|---|---|
| Semduramicin Sodium | 5.5% |
| Rice Hulls | 55.5% |
| Limestone (calcium carbonate) | 33.3% |
| Sodium Carbonate | 3.7% |
| High Viscosity Mineral Oil | 2.0% |
| FLOWABILITY (metal funnel), 11.76 g/sec/cm² (0.168 lb/sec/in²) (3-day HEUBACH DUSTINESS VALUE (µg drug/membrane): 720 | |

### EXAMPLE 16

| | |
|---|---|
| Semduramicin Sodium | 5.5% |
| Rice Hulls | 53.1% |
| Limestone (calcium carbonate) | 31.86% |
| Sodium Carbonate | 3.54% |
| High Viscosity Mineral Oil | 6.0% |
| FLOWABILITY (metal funnel); no flow (initial) HEUBACH DUSTINESS VALUE (µg drug/membrane): <0.1 | |

### EXAMPLE 17

| | |
|---|---|
| Semduramicin Sodium | 5.5% |
| Rice Hulls | 51.9% |
| Limestone (calcium carbonate) | 31.14% |
| Sodium Carbonate | 3.46% |
| High Viscosity Mineral Oil | 8.0% |
| FLOWABILITY (metal funnel), : no flow (initial) HEUBACH DUSTINESS VALUE (µg drug/membrane): <0.1 | |

### EXAMPLE 18

| | |
|---|---|
| Semduramicin Sodium | 5.5% |
| Rice Hulls | 50.8% |
| Limestone (calcium carbonate) | 33.2% |
| Sodium Carbonate | 4.0% |
| High Viscosity Mineral Oil | 4.0% |
| Sodium Aluminosilicate | 2.5% |
| FLOWABILITY (metal funnel): 11.9 g/sec/cm² (0.17 lb/sec/in²) (initial HEUBACH DUSTINESS VALUE (µg drug/membrane): 164 | |

## Claims

1. A Semduramicin animal premix comprising:
a. 2 weight % to 10 weight % Semduramicin or a pharmaceutically acceptable cationic salt thereof;
b. 0.5 weight % to 50 weight % Semduramicin degradation reducing stabilizer;
c. 40 weight % to 80 weight % diluent;
d. 5 weight % to 50 weight % density increasing bulking agent;
e. 2 weight % to 10 weight % dust controlling oil; and
f. 0.25 weight % to 5 weight % flowability enhancing glidant selected from the group consisting of sodium aluminosilicate and silicon dioxide.

2. A premix as recited in claim 1 wherein said stabilizer is a monovalent basic or neutral salt; said diluent is grain by-products; said bulking agent is limestone or sodium carbonate; and said oil is mineral oil.

3. The premix as recited in claim 2 wherein said stabilizer is sodium carbonate; said diluent is rice hulls; said bulking agent is sodium carbonate; said oil is low density oil and said glidant is sodium aluminosilicate.

4. The premix as recited in claim 3 wherein said premix contains 30 weight % to 40 weight % sodium carbonate; 45 weight % to 55 weight % diluent; 4 weight % to 6.5 weight % oil; and 1 weight % glidant.

5. The premix as recited in claim 2 wherein said stabilizer is sodium carbonate; said diluent is rice hulls; said bulking agent is limestone; said oil is high density oil; and said glidant is sodium aluminosilicate.

6. The premix as recited in claim 5 wherein said premix contains 3 weight % to 6 weight % stabilizer; 45 weight % to 55 weight % diluent; about 30 weight % to 40 weight % bulking agent; 4 weight % to 6.5 weight % oil and 2 weight % to 3 weight % glidant.

7. An animal feed comprising an antibacterial effective amount of the premix of claim 1 and animal feed.

8. The animal feed as recited in claim 7 comprising 0,45 kg (1 pound) of the premix of claim 1 per 900 kg (1 ton) of animal feed.

9. A process for preparing a Semduramicin premix comprising: forming a mixture comprising a diluent, a density increasing bulking agent, a glidant, a dust controlling oil, a Semduramicin degradation reducing stabilizer and Semduramicin or a pharmaceutically acceptable salt of Semduramicin; wherein the amount of said diluent is 40 weight % to 80 weight %, the amount of said bulking agent is 5 weight % to 50 weight %, the amount of said glidant is 0.25 weight % to 5 weight %, the amount of said oil is 2 weight % to 10 weight %, the amount of said stabilizer is 0.5 weight % to 50 weight % and the amount of said Semduramicin or pharmaceutically acceptable salt thereof is 2 weight % to 10 weight % of the final concentration of the premix.

## Patentansprüche

1. Semduramicintiervormischung, umfassend:
a. 2 bis 10 Gew.-% Semduramicin oder ein pharmazeutisch akzeptables kationisches Salz hiervon;
b. 0,5 bis 50 Gew.-% eines einen Semduramicinabbau verringernden Stabilisators;
c. 40 bis 80 Gew.-% Verdünnungsmittel;
d. 5 bis 50 Gew.-% die Dichte erhöhendes Masse lieferndes Mittel;
e. 2 bis 10 Gew.-% die Staubbildung steuerndes Öl und
f. 0,25 bis 5 Gew.-% die Fließfähigkeit erhöhendes Gleitmittel, ausgewählt aus der Gruppe Natriumalumosilicat und Siliciumdioxid.

2. Vormischung nach Anspruch 1, wobei der Stabilisator aus einem einwertigen basischen oder neutralen Salz, das Verdünnungsmittel aus Kornnebenprodukten, das Masse liefernde Mittel aus Kalkstein oder Natriumcarbonat und das Öl aus Mineralöl bestehen.

3. Vormischung nach Anspruch 2, wobei der Stabilisator aus Natriumcarbonat, das Verdünnungsmittel aus Reishülsen, das Masse liefernde Mittel aus Natriumcarbonat, das Öl aus niedrigdichtem Öl und das Gleitmittel aus Natriumalumosilicat bestehen.

4. Vormischung nach Anspruch 3, wobei die Vormischung 30 bis 40 Gew.-% Natriumcarbonat, 45 bis 55 Gew.-% Verdünnungsmittel, 4 bis 6,5 Gew.-% Öl und 1 Gew.-% Gleitmittel enthält.

5. Vormischung nach Anspruch 2, wobei der Stabilisator aus Natriumcarbonat, das Verdünnungsmittel aus Reishülsen, das Masse liefernde Mittel aus Kalkstein, das Öl aus hochdichtem Öl und das Gleitmittel aus Natriumalumosilicat bestehen.

6. Vormischung nach Anspruch 5, wobei die Vormischung 3 bis 6 Gew.-% Stabilisator, 45 bis 55 Gew.-% Verdünnungsmittel, etwa 30 bis 40 Gew.-% Masse lieferndes Mittel, 4 bis 6,5 Gew.-% Öl und 2 bis 3 Gew.-% Gleitmittel enthält.

7. Tierfutter mit einer antibakteriell wirksamen Menge der Vormischung nach Anspruch 1 und Tierfutter.

8. Tierfutter nach Anspruch 7, umfassend 0,45 kg (1 Pound) der Vormischung nach Anspruch 1 pro 900 kg (1 ton) Tierfutter.

9. Verfahren zur Herstellung einer Semduramicinvormischung durch
Ausformen eines ein Verdünnungsmittel, ein die Dichte erhöhendes Masse lieferndes Mittel, ein Gleitmittel, ein die Staubbildung steuerndes Öl, einen einen Semduramicinabbau verringernden Stabilisator und Semduramicin oder ein pharmazeutisch akzeptables Salz von Semduramicin umfassenden Gemisches, wobei die Menge des Verdünnungsmittels 40 bis 80 Gew.-%, die Menge des Masse liefernden Mittels 5 bis 50 Gew.-%, die Menge des Gleitmittels 0,25 bis 5 Gew.-%, die Menge des Öls 2 bis 10 Gew.-%, die Menge des Stabilisators 0,5 bis 50 Gew.-% und die Menge des Semduramicins oder des pharmazeutisch akzeptablen Salzes hiervon 2 bis 10 Gew.-% der Endkonzentration der Vormischung betragen.

## Revendications

1. Prémélange pour animaux contenant de la Semduramicine, qui comprend :
a. 2 % en poids à 10 % en poids de Semduramicine ou d'un sel cationique acceptable du point de vue pharmaceutique de ce composé ;
b. 0,5 % en poids à 50 % en poids d'agent stabilisant réduisant la dégradation de la Semduramicine ;
c. 40 % en poids à 80 % en poids de diluant ;
d. 5 % en poids à 50 % en poids de charge élevant la densité ;
e. 2 % en poids à 10 % en poids d'huile limitant la formation de poussière ; et
f. 0,25 % en poids à 5 % en poids d'agent de glissement améliorant l'aptitude à l'écoulement, choisi dans le groupe constitué de l'aluminosilicate de sodium et du dioxyde de silicium.

2. Prémélange suivant la revendication 1, dans lequel l'agent stabilisant est un sel monovalent basique ou neutre ; le diluant est un sous-produit de céréales ; la charge est du calcaire ou du carbonate de sodium ; et l'huile est une huile minérale.

3. Prémélange suivant la revendication 2, dans lequel l'agent stabilisant est le carbonate de sodium ; le diluant est formé de balles de riz ; la charge est du carbonate de sodium ; l'huile est une huile de faible masse volumique et l'agent de glissement est l'aluminosilicate de sodium.

4. Prémélange suivant la revendication 3, qui contient 30 % en poids à 40 % en poids de carbonate de sodium ; 45 % en poids à 55 % en poids de diluant ; 4 % en poids à 6,5 % en poids d'huile ; et 1 % en poids d'agent de glissement.

5. Prémélange suivant la revendication 2, dans lequel l'agent stabilisant est le carbonate de sodium ; le diluant est formé de balles de riz ; la charge est du calcaire ; l'huile est une huile de haute masse volumique ; et l'agent de glissement est l'aluminosilicate de sodium.

6. Prémélange suivant la revendication 5, qui contient 3 % en poids à 6 % en poids d'agent stabilisant ; 45 % en poids à 55 % en poids de diluant ; environ 30 % en poids à 40 % en poids de charge ; 4 % en poids à 6,5 % en poids d'huile et 2 % en poids à 3 % en poids d'agent de glissement.

7. Aliment pour le bétail, comprenant une quantité à effet antibactérien du prémélange suivant la revendication 1 et un aliment pour le bétail.

8. Aliment pour le bétail suivant la revendication 7, comprenant 0,45 kg (1 lb) du prémélange suivant la revendication 1 pour 900 kg (1 short ton) d'aliment pour le bétail.

9. Procédé de production d'un prémélange contenant de la Semduramicine, qui comprend : la formation d'un mélange comprenant un diluant, une charge élevant la masse volumique, un agent de glissement, une huile limitant la formation de poussière, un agent réduisant la dégradation de la Semduramicine, et de la Semduramicine ou un sel de Semduramicine acceptable du point de vue pharmaceutique ; dans lequel la quantité de diluant est de 40 % en poids à 80 % en poids, la quantité de charge est de 5 % en poids à 50 % en poids, la quantité d'agent de glissement est de 0,25 % en poids à 5 % en poids, la quantité d'huile est de 2 % en poids à 10 %, la quantité d'agent stabilisant est de 0,5 % en poids à 50 % en poids et la quantité de Semduramicine ou de sel pharmaceutiquement acceptable de Semduramicine est de 2 % en poids à 10 % en poids de la concentration finale du prémélange.
